Europäisches Patentamt

(19) European Patent Office

Office européen des brevets

(11) Publication number: **0 437 013 A2**

(12) # EUROPEAN PATENT APPLICATION

(21) Application number: **90303864.4**

(22) Date of filing: **10.04.90**

(51) Int. Cl.⁵: **G01N 33/58**, G01N 33/92, G01N 33/533, G01N 33/543

(30) Priority: **10.04.89 US 335826**

(43) Date of publication of application:
**17.07.91 Bulletin 91/29**

(84) Designated Contracting States:
**AT BE CH DE DK ES FR GB GR IT LI LU NL SE**

(71) Applicant: **ELA TECHNOLOGIES INC.**
**187 Benburton Circle**
**Bogart, Georgia 30622 (US)**

(72) Inventor: **Smith, David F.**
**205 Pendleton Drive**
**Athens, Georgia 30604 (US)**
Inventor: **McCann, Richard O**
**180 Longview Drive**
**Athens, Georgia 30605 (US)**

(74) Representative: **Armitage, Ian Michael et al**
**MEWBURN ELLIS 2 Cursitor Street**
**London EC4A 1BQ (GB)**

(54) **Luminescent assays.**

(57) An improvement for a method for detecting the presence of an analyte in a sample by the interaction of the analyte with a specific binding compound to form a complex in a reaction medium, in which formation of the complex is detected by measurement of light emitted by a luminescent molecule used as a label on a component of the specific binding system that forms the complex, is provided in which the improvement comprises using as the label a photoprotein bound to a luminescent molecule, wherein the luminescent molecule remains bound to the photoprotein without emitting light in the absence of an external trigger; reducing background light emission in the reaction medium by contacting the reaction medium with the apophotoprotein form of the photoprotein prior to introduction of the photoprotein label to the reaction medium; and adding the trigger to the reaction medium after formation of the complex.

This invention relates to luminescent assays and to methods for increasing the sensitivity of such assays.

A wide variety of analytical techniques have been devised using specific binding compounds to interact with analytes to form a complex. Such techniques can broadly be divided into heterogeneous assays, in which the specific-binding-compound/analyte complex is separated from unreacted analyte and binding compound, and homogeneous assays, in which no such separation is required. One or more signal-generating labels, attached to one or more of the components of the binding system, are used to indicate the formation of a complex.

These techniques were first developed using radioactive isotopes as labels, as exemplified by radioimmunoassays, first commonly used in the 1960's. In a competitive radioimmunoassay, as illustrated in equation 1 below, the assay is based on competition between the target analyte (an antigen ; Ag) and a known amount of a labelled antigen (Ag*) for a limited number of antibody binding sites on the specific binding compound (antibody, Ab), resulting in the partitioning of the label between free and bound states. Either the free or bound labelled antigen can then be measured, allowing quantitation of antigen.

Equation 1 (non-stoichiometric)
$$Ag + Ag^* + Ab \rightarrow Ab\text{-}Ag^* + Ab\text{-}Ag + Ag^* + Ag$$

Another example of a specific binding assay is the increasingly popular sandwich assay, in which two specific binding compounds interact with a single analyte molecule (e.g., $Ab_1$ - Ag - $Ab_2$). Typically one of the binding compounds is attached to a solid surface and is used to capture the analyte onto that surface. A second binding compound, labelled with a detectable label, then binds with a second binding site on the analyte so that the label is captured onto the solid surface for easy measurement. Sandwich assays are also referred to as immunometric assays. Numerous other assay types that utilize specific binding are known, as exemplified by the assays described in the publications cited in this specification.

In addition to radioactive labels, numerous other labels have been used in specific bindings assays. In many cases, fluorescent molecules and enzymes have been used to avoid the disposal problems resulting from the use of radioactive labels. Luminescent molecules have also been proposed as labels. Both chemiluminescent and bioluminescent compounds are available. A chemiluminescent compound is one which, under appropriate conditions (usually oxidation) produces a fluorescent product in the singlet electronic excited state. Decay to the ground state produces a photon of light. Examples of chemiluminescent molecules include luminol, isoluminol, diacridinium salts, iophine, and oxalate esters. Since the emission of visible light is easily measured and quantitated, chemiluminescent labels have been successfully used in certain types of immunoassays. Examples of chemiluminescent assays are disclosed in U.S. Patent No. 4,220,450, U.S. Patent No. 4,104,029 and United Kingdom Patent No. 1,461,877. Recent progress in the field of chemiluminescent immunoassays is also summarized in "Luminescent Assays," N. Serio and M. Pazzagli eds., Vol. 1, Ravin Press, 1982.

Homogeneous chemiluminescent immunoassays have been described based on energy transfer (Patel et al., Bio. Chem. Soc. Trans., 1983, 11 :196). Aminobutylethylisoluminol was coupled to an antigen as a label, and the corresponding antibody was labelled with an energy transfer acceptor, a fluorescent molecule such as fluorescein. When the labeled antigens are bound to their respective fluorescein-labelled antibodies, a shift is observed in the ratio of emmission at 460nm (blue) to that at 525nm (green). This energy transfer process was used to develop homogeneous chemiluminescent assays for several antigens.

In addition to chemiluminescent molecules, bioluminescent molecules can also be used as labels. Chemiluminescent and bioluminescent molecules are distinguished by the requirement for a protein interaction in bioluminescent assays, generally either a light-triggering enzyme such as a luciferase or a photoprotein that binds the actual luminescent molecule but does not produce light in the absence of an external trigger, such as calcium ions. A number of such assays are described in chapter 5 of "Practical Immunoassay," edited by Wilfrid R. Vutt, Vol. 14 Clinical and Biochemical Assays, Marcel Dekkar, Inc., 1984, and by Patel and Cormier, Analytical Applications of Bioluminescense and Chemiluminescence, pp. 273-276, Academic Press, Inc., 1984. Also see U.S. Patent No. 478,817, entitled "Detecting or Quantifying Substances Using Labelling Techniques," which is related to bioluminescence and chemiluminescence assays, and U.S. Application Serial No.059,137, filed June 5, 1987 (equivalent to European publication No. EP-A-137515) entitled "Bioluminescent Immunoassays Utilizing Coclenterate-Derived Luciferases and Photoproteins."

An advantage of the bioluminescent assay is the high theoretical sensitivity that is available for such compounds coupled with the lack of disposal problems. Detection of bioluminescent labels at a concentration of $10^{-20}M$ is theoretically possible. However, this theoretical limit cannot normally be approached because of, among other reasons, non-specific binding of the proteins found in bioluminescent assays to surfaces in the reaction vessel in which the analysis is being carried out as well as non-specific binding interactions between these protenacious molecules and other components that are present in the reaction medium.

The "stickiness" of proteins is well known, particularly to glass surfaces. Prior to the present invention, unrelated proteins, such as serum albumin, were used to pre-coat reaction vessel surfaces so that the proteins used in the assay would not stick to the surfaces. However, such approaches did not allow maximum sensitivity, since the binding affinities of the coating proteins for various surfaces and components of the reaction medium differed from the affinities of the label proteins.

Accordingly, there remains a need for improved techniques of using bioluminescent labels in detecting the presence of analytes in samples.

The present invention seeks to provide a method for increasing sensitivity of bioluminescence assays which ameliorates any of the above or other problems.

The invention seeks to reduce background luminescence in all types of bioluminescence assays.

This has been accomplished by providing a technique that can be used to improve the sensitivity of bioluminescence methods for detecting the presence of an analyte in a sample. Thus the present invention provides a method for detecting the presence of an analyte in a sample by the interaction of the analyte with a specific binding compound to form a complex in a reaction medium and in which formation of the complex is detected by measurement of light emitted by a luminescent molecule used as a label on a component of the specific binding system that forms the complex, which comprises :

   a. using as said label a photoprotein bound to a luminescent molecule, wherein said luminescent molecule remains bound to said photoprotein without emitting light in the absence of an external trigger ;

   b. contacting said reaction medium with an apophotoprotein of said photoprotein, wherein said apophotoprotein comprises said photoprotein in the absence of said luminescent molecule, prior to introduction of said photoprotein label to said reaction medium ; and

   c. adding said trigger to said reaction medium after formation of said complex.

The method uses a photoprotein bound to a luminescent molecule as the label on the component of the binding system that is being detected and detects light emitted by the luminescent molecule bound to the photoprotein. The photoprotein has specific properties, namely ability to bind a luminescent molecule without emitting light in the absence of an external trigger. Background light emission is reduced in the reaction medium by contacting the reaction medium with the photoprotein that contains no bound luminescent molecule (such a protein being generally referred to as an apophotoprotein) prior to introduction of trigger to the reaction medium and preferably before the label is introduced to the reaction medium. The external trigger is then added to the reaction medium to trigger light emission. Since background emission previously caused by sticking of the protein component of the bioluminescent system to surface or components of the reaction medium is prevented by the same protein that is used as the label, sensitivity is increased.

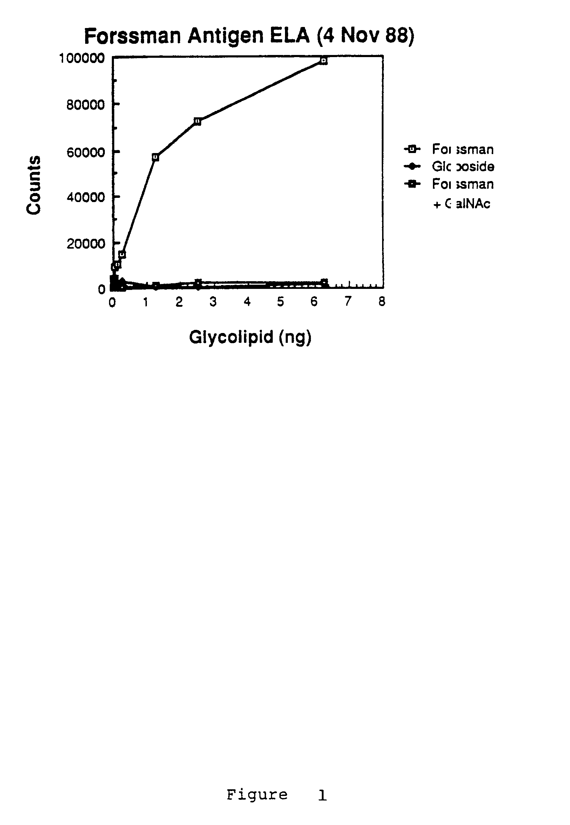

In order that the invention is more clearly understood specific embodiments will now be described in more detail (by way of example only and not by way of limitation) with reference to Figure 1 which is a graph showing light emission in the presence and absence of analyte (i.e desired light emission and background).

The present invention provides a method for increasing sensitivity of bioluminescence assays used to detect the presence and/or amount of an analyte in a sample. One of the advantages of the present invention is that it can be used to reduce background light emission in any type of bioluminescent assay, whether heterogeneous or homogeneous, direct or competitive, or any other variation. This is possible because the key steps relating to reducing background light emission are not steps that are involved in the assay itself. Instead, background light emission is reduced by contacting the reaction medium with an apophotoprotein while using the corresponding photoprotein with its bound luminescent molecule as the bioluminescence label. Suprisingly, it has been found that assays can be carried out without exchange of the actual luminescent molecule between the photoprotein label and the apophotoprotein background blocker, which would have increased (rather than decreased) background emission.

A key component of the invention is the photoprotein that is bound to its luminsecent molecule and is capable of being triggered by an external trigger for use as the detectable label for the component being detected. A corresponding apophotoprotein (the photoprotein without the bound luminescent molecule) must also exist. This photoprotein is not the same as a luciferase, a molecule that has been commonly reported for use in bioluminescence assays. Luciferases and photoproteins both catalyze the production of light from luminescent molecules but operate by different mechanisms. Luciferases normally do not bind the luminescent molecule for significant period of time prior to the oxidation reaction but instead react immediately with luciferin (in the presence of oxygen) to produce light.

The particular bioluminesence label (photoprotein) used in the method of the present invention comprises a protein or related molecule (e.g., glycoprotein) capable of binding to a luciferin or other light emitting molecule and being triggered by an external trigger. The photoproteins are readily distinguishable from luciferases and other enzymes that trigger luminescent reactions upon initial binding by their ability to bind the actual luminescent molecule in the presence of oxygen without emitting light. Luciferases and other enzymes that produce light in the presence of oxygen without requiring the use of another trigger molecule are specifically excluded from the definition of photoproteins.

Molecules that bind luciferin and related light-emitting molecules without immediately emitting light are generally called photoproteins. Coelenterate-derived photoproteins are particularly useful for the practice of the present invention. Examples of coelenterate-derived photoproteins are aequorin, obelin, mnemiopsin, and berovin. These molecules are described in a number of technical publications, including Ward et al., Proc. Natl. Acad. Sci. USA (1975) 72 : 2530-2534. Such coelenterate-derived proteins are stable in the absence of free calcium ions and contain both coelenterate luciferin and oxygen bound to the protein. When calcium ions, a bioluminescence trigger for coelenterate-derived photoproteins, are added to solutions labeled with the photoprotein, a blue light is produced having a maximum wave length of about 469 nm. Such labels are described in detail in U.S. Application Serial No. 059,137, filed June 5, 1987. A form of the apophotoprotein prepared by techniques of genetic engineering is described in U.S. Applications Serial Nos. 165,422, filed February 29, 1988, and 173,045, filed March 17, 1988, both of which are entitled "Recombinant DNA Vectors Capable of Expressing Apoaequorin," (both equivalent to European publication No. EP-A-187519).

Examples of publications that describe attachment of bioluminescent labels to analytes and other components of specific binding systems include U.S. Application Serial No. 059,137, filed June 5, 1987 entitled "Bioluminescent Immunoassays Utilizing Coelenterate-Derived Luciferases and Photoproteins" and U.S. Patent No. 4,478,817 entitled "Detecting or Quantifying Substances Using Labelling Techniques."

The conditions under which loading and unloading of the luminescent molecule onto and off of the apophotoprotein are now known, and it is possible to select conditions for the reaction medium so that no exchange of the luminescent molecule between photoprotein and apophotoprotein occurs. Under these circumstances, only photoproteins used as labels will emit light when triggered by their external trigger (calcium ions for coelenterate-derived photoproteins) since the apophotoproteins used to eliminate background luminescence do not contain a luminescent molecule.

Luminescent molecules (e.g., luciferins) can readily be bound to apophotoproteins to form photoproteins. This process is generally referred to as charging (or loading) of the photoprotein. Charging takes place under physiological conditions so that the apophotoprotein retains its normal confirmation and in the presence of a thiol-containing compound, typically a reducing agent, such as mercaptoethanol (ME) or dithiothretiol (DTT). Otherwise, there are no particular limitations on the charging conditions. Normally charging will take place between 4 and 40°C, preferably between 10 and 30°C, and more preferably at about 20-25°C, in an aqeuous solution. Buffers can be used but are not required. Normally the pH of the charging medium is between 5 and 10, preferably between 6 and 8. The luminescent molecule is typically present at a concentration greater than that of the apophotoprotein in order to insure rapid charging. Concentration ratios of luminescent molecule to photoprotein are typically in the range of from 1 :1 to 20 :1, preferably 2 :1 to 5 :1.

The thiol-containing reagent is typically a small organic molecule with a molecular weight of less than 500 in order to allow easy separation of the thiol from the charged photoprotein. Separation can be carried out by chromatography, dialysis, or other techniques that allow separation of compounds according to size. The thiol-containing compound is usually present at a concentration higher than that of the apophotoprotein.

Since the concentration of the apophotoprotein in the charging solution is usually not measured, thiol concentrations in the range of 1-10 mM are typically used as starting concentrations and can be adjusted upwards or downward as necessary to achieve the desired result (efficient charging). It has been found that no significant exchange of luminescent molecules occurs between apophotoproteins and photoproteins in the absence of soluble thiols. Accordingly, assays can be carried out in the substantial absence of thiol-containing compounds, and no transfer of luminescent molecules from photoproteins to apophotoproteins will occur. Background luminescence can therefore be eliminated by first contacting the reaction medium with the apophotoprotein and then carrying out the reaction using an already charged photoprotein in the substantial absence.

The steps involved in using the apophotoprotein to eliminate background luminesence are the same as those which have previously been used using other proteins, such as serum albumin. Typically, a dilute solution of the apophotoprotein, preferably about .01 to about 10 mM, more preferably about 0.1 to 1 mM, is used. Most prior techniques used to reduce binding with surfaces of the reaction vessel in which the reaction medium is to be placed involve coating the surfaces of the reaction vessel with the non-interfering protein solution followed by draining to remove excess liquid. This technique or any other technique for contacting the surfaces of the reaction vessel with the apophotoprotein can be carried out. Additionally, it is possible to add the apophotoprotein directly to the reaction medium solution in order to prevent any nonspecific binding between the photoprotein label and other molecules, particularly other macromolecules, such as antibodies, that are present in the reaction medium. An excess of the apophotoprotein can be provided to virtually eliminate such binding without fear of transfer of the luminescent molecule from the photoprotein to the apophotoprotein. Since the reaction medium in an analytical solution is typically under the control of the analyst, thiols can be eliminated by not adding them to the reaction medium. If thiols are known to be present in the sample, steps can be taken to eliminate thiols from the reaction medium. For example, if the analyte is a macromolecule, dialysis can be used to remove small, soluble thiol-containing compounds. If the analyte is a small molecule that would also be removed by dialysis, oxygen can be bubbled through the sample to react with and eliminate thiols.

In addition, there is no evidence to suggest that thiols influence the activity of aequorin. Although thiols are required for charging, once charged the luciferin molecule is not displaced from aequorin by thiols under the assay conditions describes.

The sample can be any sample suspected of containing the analyte being analyzed and will be handled in accordance with standard procedures relating to the type of sample being collected. Typical samples include blood (either as serum, plasma, or whole blood), urine, saliva, feces, tissue samples, and the like. The sample can be used either in the form in which it is originally obtained or it can be subjected to various processing steps to provide the anayte in a form suitable for assay. For example, if the analyte is normally found in the cytoplasm of cells, the cell walls can be disrupted to release the analyte into the reaction medium.

Once the analytical binding interaction has taken place, steps are taken to detect the presence or amount of detectable label in either the complexed or uncomplexed labelled component of the binding system. Because of the wide variety of analysis types to which the present technique may be applied, these measurements can vary widely. For example, if the assay is a homogeneous assay, quenching can be used to distinguish labeled analyte that is complexed with a quencher-tagged specific binding molecule from labeled that is present in solution. If the reaction is a heterogeneous assay, in which the solid and liquid phases are seperated, then separation of the reaction medium from the solid-phase receptor complex allows measurement of the label in either the solid-phase complex or the liquid-phase, uncomplexed, detectably labeled component. The specific steps that will be necessary for determining the presence or amount of the detectable label and one of the two components will be readily apparent to one skilled in clinical chemistry from the type of label being used and the specific type of assay (e.g., homogeneous or heterogeneous, measurement of product or unreacted substrate). Luminescence is measured after adding an appropriate trigger to the medium being measured in order to induce luminescense to occur, as is discussed above.

The individual steps of the actual binding assay can vary widely in accordance with the different techniques that are available for carrying out heterogeneous and homogeneous assays. Since the assay is not part of the present invention, there is no need to describe the variety of available assays. See the publications listed in the Background section of this specification for examples of bioluminescence assays.

The following example is offered by way of illustration and not by way of limitation.

Example

Detection of Glycolipids on Microtiter Plates

The present invention was used to reduce background emission and increase sensitivity in an analytical technique that detects glycolipids bound to a solid surface by a lectin.

The lectin from Helix pomatia was used as the specific binding compound in an assay for the glycolipid. The lectin is commercially available (Sigma Chemical Co.), and its specificity is well-documented (for recent studies see Torres and Smith, Arch. Biochem. Biophys. (1988) 262 : 1-11). The Forssman glycolipid and the human blood group assays are available in complex mixtures total lipid extracts of sheep or human erythrocytes, respectively. The purified glycolipids were obtained in a single affinity chromatographic step from the crude extracts of these erythrocyte membranes (Torres and Smith, 1988).

The primary reagents in the assay were Helix pomatia lectin, recombinant aequorin, and streptavidin. Native aequorin is a 20,000 dalton bioluminescent protein from the jellyfish Aequorea victoria. Each molecule of protein contains 1 molecule of the coelenterate-type chromophore, luciferin, when charged (Shimomura, et al., J. Cell. Comp. Physiol. (1962) 59: 223-238 ; Ward and Cormier, Proc. Natl. Acad. Sci. USA (1975) 72 : 2530-2534). The protein produces blue light ($\lambda$ max$^{=469nm}$) in the presence of Ca$^{++}$. The quantum yield is such that aequorin is detectable in the range of $10^{-19}$ to $10^{-20}$ M. Aequorin has been cloned as previously described, and gram quantities of expressed aequorin, whose light producing properties are essentially identical to native aequorin, are available. Biotinylated aequorin (B-AEQ) was prepared using NHS-LC biotin from Pierce Chemical Company. Apoaequorin was dissolved in 0.1 M NaHCO$_3$, 0.2 M NaCl, pH 8.4, at a concentration of approximately 1.0 mg/ml. The NHS-LC biotin derivative was then added to give a 3-fold molar excess of biotin over apoaequorin, and the mixture was incubated at 4°C for four hours. The reaction was stopped by adding 25 $\mu$l of 1 M glycine to 0.1 M NaHCO$_3$, pH 8. Reaction side-products were separated from biotinylated apoaequorin on a Sphadex G-25 column (Pharmacia PD-10 or equivalent), and the biotinylated protein was stored at 4° C in 50 mM Tris, 0.2 M NaCl, 5 mM EDTA, pH 8.

Streptavidin is a 60,000 dalton biotin-binding protein from Streptomyces avidinii. Each molecule of the protein can bind 4 mols of biotin (Chalet and Wolf, Arch. Biochem. Biophys. (1964) 106 : 1). Streptavidin, which is commercially available, binds with high affinity to biotin ($K_D$ approximately $10^{-15}$M).

The biotinylated Helix pomatia agglutinin (B-HPA) was prepared by dissolving 5 mg of the pure protein (Sigma Chemical Co.) in 2 ml of biotinylation buffer (0.1 M sodium borate, pH 8.5) and biotinylated by adding a 3X molar excess of NHS-LC-Biotin (Pierce) and incubating for 1 hr at room temperature. The product of this reaction was dialyzed against phosophate-buffered saline containing 5 mM EDTA (PBS-EDTA) and stored at -80°. Other reagents were obtained commercially.

The glycolipids were dissolved in methanol, and aliquots (50 μl) of dilutions containing 0.001 to 25 ng of glycolipid were added to wells of microtiter plates (Falcon #3911). The methanol was allowed to evaporate overnight at room temperature to bind the glycolipids to the surface of the microtiter plate well. Binding and washing steps for detecting the Forssman glycolipid were subsequently performed in the following order :

1) To prevent non-specific binding of the biotinylated aequorin, the wells were treated with a solution of a apoaequorin (1 mg/ml) in PBS-EDTA for 30 min and subsequently washed with PBS-EDTA.
2) B-HPA (0.1 mg/ml in PBS-EDTA) was added and incubated for 30 min.
3) Wash with PBS-EDTA.
4) Incubate 30 min with streptavidin (SA ; 0.1 mg/ml in PBS-EDTA).
5) Wash with PBS-EDTA.
6) Incubate 30 min with B-AEQ (1 μg/ml in PBS-EDTA).
7) Wash with PBS-EDTA.

The volumes of solutions used for incubations and washings were 200 μl, and steps 5-7 were carried out at 4° C. After step 7 the microtiter wells contain the ternary complex glycolypid-(B-HPA)-SA-(B-AEQ). The bound B-AEQ was eluted by a 30 min incubation of wells with PBS-EDTA containing 25 mM GalNAc. An aliquot of the eluate which contained the B-AEQ that was bound to the glycolipid was quantified as photons after the addition of Ca$^{++}$ in a Berthold luminometer Model 9500. The results of this Experiment are shown in Figure 1. The assay is specific for the Forssman glycolipid ; i.e., unreactive with globoside, the immediate precursor to the Forssman structure with a terminal β1,3-linked GalNAc, and the reaction is completely inhibited when GalNAc is included in the reaction mixture.

In this assay the reliable limit of detection for the Forssman glycolipid in the microtiter well is 62.5 picograms (42 femtomol). These results confirm our ability to detect Forssman glycolipid at the femtomol level with a prototype assay requiring elution of the aequorin from the ternary complex prior to its measurement. A similar assay carried out without using apoaequorin as a blocking agent had a detection limit of 1000 picograms.

All publications and patent applications cited in this specification are herein incorporated by reference as if each individual publication or patent application were specifically and individually indicated to be incorporated by reference.

Although the foregoing invention has been described in some detail by way of illustration and example for purposes of clarity of understanding, it will be readily apparent to those of ordinary skill in the art in light of the teachings of this invention that certain changes and modifications may be made thereto without departing from the spirit or scope of the appended claims.

## Claims

1. A method for detecting the presence of an analyte in a sample by the interaction of the analyte with a specific binding compound to form a complex in a reaction medium and in which formation of the complex is detected by measurement of light emitted by a luminescent molecule used as a label on a component of the specific binding system that forms the complex, which comprises ;
   a. using as said label a photoprotein bound to a luminescent molecule, wherein said luminescent molecule remains bound to said photoprotein without emitting light in the absence of an external trigger ;
   b. contacting said reaction medium with an apophotoprotein of said photoprotein, wherein said apophotoprotein comprises said photoprotein in the absence of said luminescent molecule, prior to introduction of said photoprotein label to said reaction medium ; and
   c. adding said trigger to said reaction medium after formation of said complex.

2. The method of Claim 1, wherein said specific binding compound is a lectin, an antibody, or a cell surface receptor.

3. The method of Claim 1, or claim 2 wherein said specific binding compound is attached to a solid surface, whereby a solid-phase complex forms between said specific binding compound and said detectably labeled analyte.

4. The method of Claim 3, wherein said labeled component is present in a liquid-phase reaction medium and said solid-phase complex is separated from said liquid-phase reaction medium prior to said determining.

5. The method of anyone of the preceding claims wherein said photoprotein is a coelenterate photoprotein.

6. The method of anyone of claims 1 to 4, wherein said photoprotein is aequorin and said apophotoprotein is apoaequorin.

7. The method of Claim 6, wherein adding said trigger comprises adding calcium ions to said reaction medium immediately prior to said determining and said determining comprises measuring light emitted by aequorin.

Figure   1